# EUROPEAN PATENT APPLICATION

(11) **EP 3 002 006 A1**
(43) Date of publication of application: **06.04.2016**
(21) Application number: 14187289.5
(22) Date of filing: 01.10.2014
(51) Int. Cl.: A61K 9/16, A61K 9/20

(54) **Pharmaceutical composition capable for the incorporation Febuxostat in the crystalline modifications F10, II, G and A**

(71) Applicant: Bluepharma - Industria Farmacêutica, S.A., 3045-016 Coimbra (PT)
(72) Inventor: Leitão Silva, Gabriel, 3020-229 Coimbra (PT); Becker, Christian, 55217 Mainz (DE)
(74) Representative: Drescher, Christian

(57) **Abstract**

The present invention relates to pharmaceutical compositions of Febuxostat in the crystalline form F10 obtainable by wet granulation comprising a granular phase with granules and an extragranular phase wherein the wherein the percentage of the granules is 70 weight-% or more relative to the total weight of the composition, the composition being flexible towards the interchange of crystalline form F10 into other modifications of Febuxostat such as form II, form G or form A.

## Description

The present invention relates to pharmaceutical compositions of Febuxostat in the crystalline form F10 obtainable by wet granulation comprising a granular phase with granules and an extragranular phase wherein the wherein the percentage of the granules is 70 weight-% or more relative to the total weight of the composition, the composition being flexible towards the interchange of crystalline form F10 into other modifications of Febuxostat such as form II, form G or form A.

Febuxostat is the International Nonproprietary Name (INN) of Febuxostat (2-[3-cyano-4-(2-methylpropoxy)phenyl]-4-methylthiazole-5-carboxylic acid with the following chemical structure:

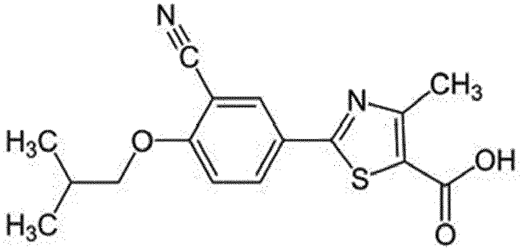

Febuxostat is a non-purine inhibitor of xanthine oxidase which is useful in decreasing serum uric acid, registered and marketed under the trade names Uloric^{®} and Adenuric^{®} used for the treatment of hyperuricaemia in patients with chronic gout.

Several crystalline modifications of Febuxostat have been described in the art. Febuxostat in the crystalline form F10 is first disclosed in WO 2010/144685. However, this application does not disclose any specific pharmaceutical formulations comprising Febuxostat form F10.

IN 277/DEU2014 reports that formulating Febuxostat in the crystalline form F10 by a dry granulation (dry compaction) process results in poor flow properties and heavy sticking. To overcome this obstacle, the application proposes Febuxostat rather complex tablet formulations containing a first minor intragranular part prepared by granulation and a additional major extragranular part. Accordingly, tablets containing micronized form F10 wherein the weight ratio of the second extragranular part to the first intragranular part is between 1:2 and 3,5:1 are suggested. However, the weight ratio of the intragranular part compared to the extragranular part still has an impact on the dissolution profile of Febuxostat, limiting the freedom of the formulator to adapt the qualitative and/or quantitative composition when necessary, e.g. in the upscaling process. In addition, a surfactant (Sodium Lauryl Sulfate) has to be added in order to enhance the solubility of the poorly soluble drug Febuxostat.

It has now been found that the pharmaceutical formulations of Febuxostat according to the present invention prepared by wet granulation processes wherein the tablets comprise more than 70% percent by weight of granules relative to the total weight of the pharmaceutical composition, exhibit a similar dissolution profile in comparison with the market products Adenuric^{®}/ Uloric^{®}.

In addition, the formulations according to the present invention exhibit improved properties with respect to stickiness and flow properties.

Accordingly, the present invention provides pharmaceutical compositions obtainable by wet granulation comprising
a) A granular phase comprising
   - from 5,0% to 30,0 % by weight Febuxostat crystalline form F10;
   - from 30,0 % to 85,0 % by weight of one or more fillers;
   - from 0,5% to 5,0% by weight of one or more binders;
   - from 1,0% to 10,0% by weight of one or more disintegrants;
   - optionally between 0,1% and 1,5% by weight of one or more glidants; and
   - optionally other acceptable pharmaceutical excipients
b) an extragranular phase comprising
   - from 0,5% to 5,0% by weight of one or more lubricants and
   - optionally other acceptable pharmaceutical excipients
wherein the percentage of the granules is 70% or more, all % by weight values being relative to the total weight of the composition.

Preferably, tablets according to the present invention comprise more than 80%, more preferably more than 90% and most preferably more than 95% percent by weight of granules relative to the total weight of the pharmaceutical composition.

As already indicated above, several crystalline modifications of Febuxostat are known. For example, Febuxostat in the crystalline form G and form A are known from patent application WO 99/65885; Febuxostat in the crystalline form II is known from patent application WO 2012/007487.

It is desirable to possess a pharmaceutical formulation of Febuxostat with some flexibility towards the exchange of the crystalline modifications therein. However, the solubilities of the crystalline modifications of Febuxostat vary at least by a factor of about 3,5 (see table 1), rendering the development of pharmaceutical compositions of Febuxostat wherein the crystalline form may be interchanged without affecting the dissolution profile of the finished dosage form into quite a challenge.

**Table 1**

| **Crystalline modification (Form)** | **Solubility (Phosphate buffer, pH 602; n=3)** |
|---|---|
| Form "F10" | 0,26 mg/ml |
| Form "II" | 0,36 mg/ml |
| Form "G" | 0,13 mg/ml |
| Form "A" | 0,10 mg/ml |

It has now been found that, in the formulations according to the present invention, Febuxostat in the crystalline form 10 may be interchanged by either crystalline form G, crystalline form II or crystalline form A without a significant change of the dissolution profile of the finished dosage form.

For certain compositions comprising relatively low amounts of disintegrant compared to the amount of binder, an impact of the compression force/hardness on the dissolution profile of the tablets appeared during the up-scaling process from 100g scale to Kg-scale. In particular, the increase of the compression force to 200N results in a somewhat sustained dissolution profile compared to the dissolution profile of the reference product.

On the other hand, it was found that if the ratio by weight of disintegrant and binder is 1,25:1 or higher, the tablet hardness may be varied between 50N and 200N without affecting the dissolution of the tablets.

Accordingly, a preferred embodiment of the present invention is represented by a pharmaceutical composition comprising Febuxostat in crystalline form F10 wherein the ratio by weight of disintegrant and binder is in the range between 10:1 and 1,25:1, preferably in the range between 8:1 and 1,5:1, more preferably in the range between 6:1 and 2:1 and most preferably in the range between 4:1 and 2,5:1.

Another embodiment of the present invention is a wet granulation process that is used to manufacture the pharmaceutical compositions according to the present invention.

The pharmaceutical compositions according to the present invention, preferably in the form of tablets, comprise a granular phase and optionally an extragranular phase. The granular phase according to the invention comprises pharmaceutical excipients like one or more fillers, one or more binders and one or more disintegrants and optionally one or more lubricants, glidants and other pharmaceutically acceptable excipients.

The extragranular phase according to the invention does typically comprise one or more lubricants and optionally one or more fillers, disintegrants, glidants as well as other pharmaceutically acceptable excipients.

Pharmaceutically acceptable fillers include, but are not limited to starch, microcrystalline cellulose (MCC), sucrose, mannitol, di calcium phosphate, calcium carbonate, magnesium carbonate, lactose, pregelatinized starch, low substituted hydroxypropyl cellulose (L-HPC), powder cellulose, calcium silicate, calcium phosphate sorbitol, dextrine, kaolin, magnesium oxide, calcium sulfate, xylitol, isomalt, glucose, fructose, maltose, acids like citric acid, tartaric acid, fumaric acid, co-polymers such as those from vinyl pyrrolidone and vinyl acetate or those of polyethylene glycol and mixtures thereof. Preferred diluents are microcrystalline cellulose, sucrose and mannitol.

The amount of filler(s) is preferably in the range between 30 wt.-% and 85 wt.-%. It has been found that high amounts of lactose might have some impact on the dissolution when increased compression force is applied. Accordingly, the amount of lactose is preferably 30 wt.-% or less.

Pharmaceutically acceptable disintegrants include, but are not limited to sodium starch glycolate, maize starch, corn starch, potato starch, polyvinylpyrrolidone (crospovidone), croscarmellose sodium, low substituted hydroxypropyl cellulose (L-HPC) and mixtures thereof. Preferred disintegrant is croscarmellose sodium.

It was found that amounts of disintegrant below 0,5 wt.-% result in poor dissolution of Febuxostat. Generally there is no upper limit, but amounts of disintegrant(s) exceeding 10 wt.-% often result in stability problems due to the high water uptake of the hygroscopic excipient(s). Accordingly, the amount of disintegrant(s) is preferably in the range between 0,5 wt.-% and 10,0 wt.-%.

Pharmaceutically acceptable binders include, but are not limited to hydroxypropyl methylcellulose (HPMC), dihydroxy propylcellulose, methyl cellulose, hydroxyethyl cellulose, ethyl cellulose, sodium carboxyl methylcellulose, polyethylene glycol, maltodextrin, pregelatinized starch, polymethacrylates, sodium alginate, polyvinylpyrrolidone (povidone) and vinylpyrrolidone/ vinylacetate copolymer (copovidone) and mixtures thereof. The preferred binder is hydroxypropyl methylcellulose (HPMC).

Generally, a minimum of 0,5 wt.-% of binder is necessary for the formation of granules, whereas it was found that amounts exceeding 5,0 wt.-% lead to poor dissolution upon application of high compression forces. Accordingly, the amount of binder(s) is preferably in the range between 0,5 wt.-% and 5,0 wt.-%.

Suitable lubricants include, but are not limited to magnesium stearate, calcium stearate, sodium stearate, stearic acid, sodium glyceryl behenate, hexanedioic acid, hygrogenated vegetable oil sodium, stearyl fumarate and glycerine fumarate and mixtures thereof. Preferred lubricant is magnesium stearate.

It has been found that a minimum of 0,5 wt.-% of lubricant is necessary to prevent stickiness. However, amounts exceeding 5 wt.-% of lubricant might have an impact on the dissolution profile of the finished dosage form. Accordingly, the amount of lubricant(s) is preferably in the range between 0,5 wt.-% and 5 wt.-%, preferably being present in the extragranular phase only.

Pharmaceutically acceptable glidants include, but are not limited to colloidal silicone dioxide, talcum, magnesium carbonate and mixtures thereof. The amount of glidant(s) is preferably in the range between 0,1 wt.-% and 1,5 wt.-%.

The terms filler(s), disintegrant(s), binder(s), lubricant(s), glidant(s) etc. shall be understood as including a single compound but also mixtures of compounds.

In a preferred embodiment of the present invention, the amount of disintegrant relatively to the amount of binder is in the range of from 10:1 to 1,25:1, preferably in the range of from 8:1 to 1,5:1, more preferably in the range of from 6:1 to 2:1 and most preferred in the range of from 4:1 to 2,5:1. In quantitative values, the amount of disintegrant is in the range from 2,0% to 7,0% by weight and the amount of binder is in the range from 1,5% to 3,0% by weight.

The preferred pharmaceutical composition is a tablet. The tablets according to the invention may further be film coated. Preferred film coating material is Opadry^{®}.

Febuxostat in the crystalline form F10 may be manufactured by known procedures, for example according to patent application WO 2010/144685. Febuxostat in the crystalline form G or form A of Febuxostat may be manufactured according to patent application WO 99/65885. Febuxostat in the crystalline form II may be manufactured according to patent application WO 2012/007487.

The wet granulation process according to the present invention comprises the following steps:
(a) The active drug is mixed with one or more fillers, one or more disintegrants and one or more glidants.
(b) Granulation liquid is added and the mixture is granulated to form wet granules;
(c) Optionally the wet granules are sieved;
(d) The sieved wet granules are dried and the dried granules are calibrated;
(e) Optionally the mixture of granules is blended with lubricant to form the final blend which is used for production of tablets
(f) The final blend is compressed into tablets which are optionally film coated

Specifically, the formulation process comprises the following steps:
(a) Febuxostat in crystalline form F10 is mixed with lactose and microcrystalline cellulose, croscarmellose sodium and colloidal silicon dioxide.
(b) A solution of HPMC in purified water is added and the mixture is granulated to form wet granules;
(c) The wet granules are passed through a sieve (1,4mm);
(d) The sieved wet granules are dried in a fluid bed drier until loss on drying is around 3%
(e) The dried granules are calibrated through a 0,710mm sieve;
(f) The mixture of granules is blended with magnesium stearate to form the final blend which is used for production of tablets
(g) The final blend is compressed into tablets
(h) The tablets are optionally film coated

The crystalline Febuxostat used in the pharmaceutical compositions exhibits a particle size with a D90 ranging from 5µm to 60µm. The particle size of the Febuxostat particles is measured by laser diffraction.

### Examples 1-5 - Pharmaceutical compositions of crystalline form of Febuxostat

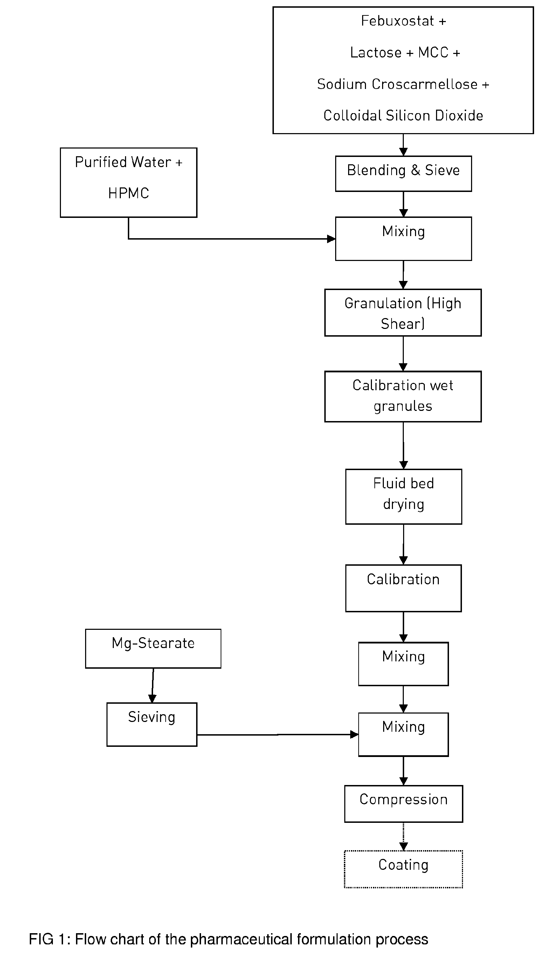

Tablets containing Febuxostat in any of the crystalline forms F10, form II, form G or form A were prepared according to the process as shown in figure 1, using equipment and settings used in the manufacturing process as described in table 2.

**Table 2**

| **Stage** | **Machine/Description** | **Settings** |
|---|---|---|
| Blending (Pre-Blend) | ERWEKA- Double Cone Mixer (lab scale) | 34 rpm |
| | Mixing time | 15min |
| | Manual Sieve | 0,71 mm |
| Granulation | Planetary granulator | 3min at 234 rpm |
| | Grid size (to sieve the wet granules) | 4,0 mm |
| Drying | Mini-Glatt | - |
| | Inlet air temperature | 37°C |
| | Air pressure | 0,5 - 1,0 bar |
| | Sieve grid size (to sieve the granulate) | 0,71 mm |
| Blending (Final Blend) | ERWEKA- Double Cone Mixer (lab scale) | 34 rpm |
| | Mixing time | 5min |
| | Sieve grid size | 0,71 mm |
| Compression | *RONCHI - Lab scale compressing machine* | - |
| Coating | Glatt - Mini- coater | - |
| | Pan: | 0,8cc |
| | Pan speed: | 4,8rpm |
| | Process air flow: | 21,8 Nm3/h |
| | Air temperature: | 43ºC |
| | Atomizing air pressure: | 0,5-0,7 bar |
| | Pattern air pressure: | 0,4-0,5 bar |
| | Spray rate: | 1,0-1,8g/min |
| | Weight gain: | 3% |

Pharmaceutical compositions with the qualitative and quantitative as displayed in table 3 were prepared:

**Table 3**

| | | **Example #1** | | **Example #2** | | **Example #3** | | **Example #4** | | **Example #5** | |
|---|---|---|---|---|---|---|---|---|---|---|---|
| Excipient | Function | Amount (mg) | Rel.* amount | Amount (mg) | Rel.* amount | Amount (mg) | Rel.* amount | Amount (mg) | Rel.* amount | Amount (mg) | Rel.* amount |
| Granular phase | | | | | | | | | | | |
| Febuxostat Form F10 | Drug substance | 80,0 | 15,4% | - | - | - | - | - | - | 80,0 | 15,4% |
| Febuxostat Form II | | - | - | 80,0 | 15,4% | - | - | - | - | - | - |
| Febuxostat Form G | | - | - | - | - | 80,0 | 15,4% | - | - | - | - |
| Febuxostat Form A | | - | - | - | - | - | - | 80,0 | 15.4% | - | - |
| Microcrystalline Cellulose | Filler | 323,0 | 62,1% | 323,0 | 62,1% | 323,0 | 62,1% | 323,0 | 62,1% | 323,0 | 62,1% |
| Lactose Monohydrate | Filler | 78,0 | 15,0% | 78,0 | 15,0% | 78,0 | 15,0% | 78,0 | 15,0% | 78,0 | 15,0% |
| HPMC | Binder | 13,0 | 2,5% | 13,0 | 2,5% | 13,0 | 2,5% | 13,0 | 2,5% | 7,8 | 1,5% |
| Croscarmellose Sodium | Disintegrant | 15,6 | 3,0% | 15,6 | 3,0% | 15,6 | 3,0% | 15,6 | 3,0% | 20,8 | 4,0% |
| Colloidal Silicon Dioxide | Glidant | 5,2 | 1,0% | 5,2 | 1,0% | 5,2 | 1,0% | 5,2 | 1,0% | 5,2 | 1,0% |
| Extragranular phase | | | | | | | | | | | |
| Magnesium Stearate | Lubricant | 5,2 | 1,0% | 5,2 | 1,0% | 5,2 | 1,0% | 5,2 | 1,0% | 5,2 | 1,0% |
| | | | | | | | | | | | |
| TOTAL | | 520 | 100% | 520 | 100% | 520 | 100% | 520 | 100% | 520 | 100% |

| | | | | | | | | | | | |
|---|---|---|---|---|---|---|---|---|---|---|---|
| *Relative amount, based on the weight of the total composition | | | | | | | | | | | |

Tablets containing 40 mg of Febuxostat may be prepared accordingly by using half the amount of drug and half the amount of excipients as indicated above. Tablets containing 120mg of Febuxostat may be prepared accordingly by using the 1,5-fold amount of the drug and the 1,5-fold amount of excipients as indicated above.

### Dissolution testing

The in-vitro dissolution of tablets according to examples 2 to 5 were analyzed according to Ph. Eur. 2.9.3 / USP <711> - Method 2 (Paddle Apparatus) with the settings as displayed in table 4.

**Table 4**

| | |
|---|---|
| Equipment: | Varian |
| Medium: | Phosphate buffer |
| Volume: | 900ml |
| Temperature: | 37,0ºC ± 0,5ºC |
| Method | UV - 247nm |
| Stirring speed: | 75 rpm |

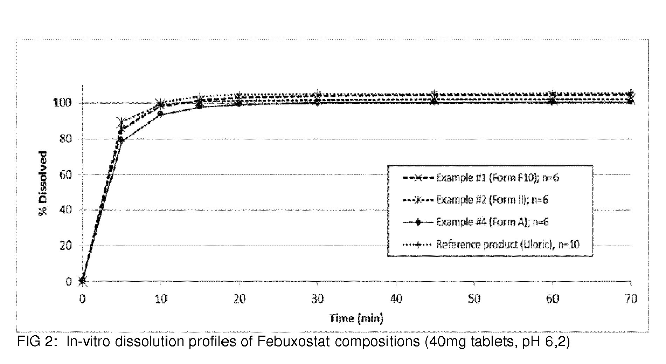

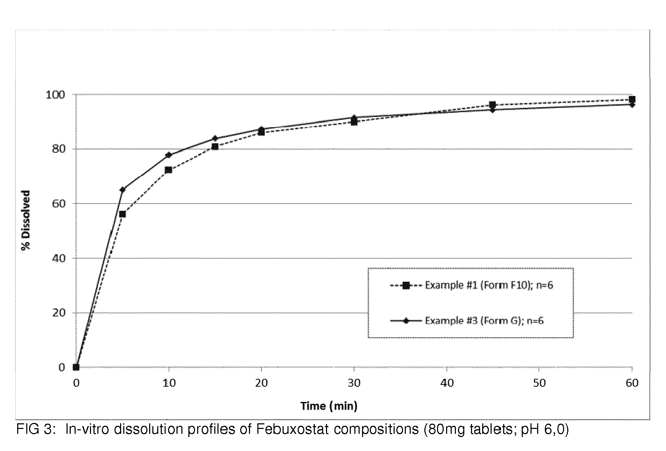

As can be seen in Figures 2 and 3, tablets being manufactured according to example 1 exhibit an almost identical dissolution profile compared to the reference product. As can further be seen in Figures 2 and 3, an interchange of crystalline form F10 by one of the crystalline forms II, form G or form A does not influence the dissolution profile significantly. This means that the excipient mixture and finished dosage form do not have to be adopted upon formulating either of the crystalline forms F10, form II, form G or form A.

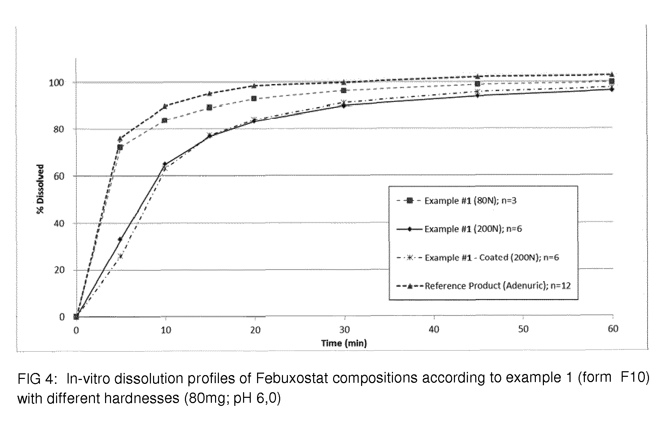

As can be seen in Figure 4, the dissolution of tablets being manufactured according to example 1 with a hardness of 200N is significantly delayed compared to tablets with a hardness of only 80N.

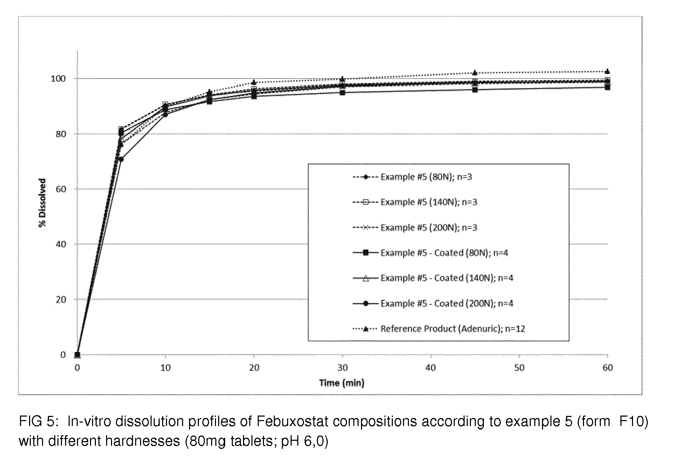

On the other hand and shown in Figure 5, the dissolution profiles of tablets being manufactured according to example 5 with a hardness between 80N and 200N remain practically identical, indicating no dependency on the compression forces applied during tableting.

As can further be deducted from Figures 4 and 5, the coating does not affect the dissolution significantly.

## Claims

1. Pharmaceutical composition obtainable by wet granulation comprising
(a) granules comprising
i. from 5,0% to 30,0% by weight Febuxostat crystalline form F10;
ii. from 30,0% to 85,0% by weight of one or more fillers;
iii. from 0,5% to 5,0% by weight of one or more binders;
iv. from 0,5% to 10,0% by weight of one or more disintegrants;
v. optionally between 0,1% and 1,5% by weight of one or more glidants and
vi. optionally other acceptable pharmaceutical excipients
(b) an extragranular phase comprising
i. from 0,5% to 5,0% by weight of one or more lubricants and
ii. optionally other acceptable pharmaceutical excipients.
wherein the percentage of the granules is 70% or more, all % by weight being relative to the total weight of the composition.

2. Pharmaceutical composition according to claim 1, wherein the percentage by weight of the granules relatively to the total weight of the composition is 80% by weight or more, preferably 90% by weight or more, most preferably 95% by weight or more.

3. Pharmaceutical composition according to any of the preceding claims, wherein the one or more fillers are selected from microcrystalline cellulose (MCC), mannitol, sucrose, lactose, di calcium phosphate, calcium carbonate, magnesium carbonate, pregelatinized starch, low substituted hydroxypropyl cellulose (L-HPC), powder cellulose, calcium silicate, calcium phosphate, sorbitol, mannitol, dextrine, kaolin, magnesium oxide, calcium sulfate, xylitol, isomalt, glucose, fructose, maltose, acids like citric acid, tartaric acid, fumaric acid and co-polymers from vinyl pyrrolidone and vinyl acetate or co-polymers of polyethylene glycol; preferably being selected from microcrystalline cellulose (MCC), lactose, mannitol and sucrose.

4. Pharmaceutical composition according to any of the preceding claims, wherein the one or more disintegrants are selected from croscarmellose sodium, sodium starch glycolate, maize starch, corn starch, potato starch, polyvinylpyrrolidone (crospovidone) and low substituted hydroxypropyl cellulose (L-HPC).

5. Pharmaceutical composition according to any of the preceding claims, wherein the one or more binders are selected from the group comprising of hydroxypropyl methylcellulose (HPMC), dihydroxy propyl cellulose, methyl cellulose, hydroxyethyl cellulose, ethyl cellulose, sodium carboxyl methylcellulose, polyethylene glycol, maltodextrin, pregelatinized starch, polymethacrylates, sodium alginate, polyvinylpyrrolidone (povidone) and vinylpyrrolidone/ vinylacetate copolymer (copovidone).

6. Pharmaceutical composition according to any of the preceding claims, wherein the one or more lubricants are selected from magnesium stearate, calcium stearate, sodium stearate, stearic acid, sodium glyceryl behenate, hexanedioic acid, hygrogenated vegetable oil sodium, stearyl fumarate and glycerine fumarate.

7. Pharmaceutical composition according to any of the preceding claims, wherein the one or more optional glidants are selected from colloidal silicone dioxide, talcum and magnesium carbonate.

8. Pharmaceutical composition according to any of the preceding claims, **characterized in that** the ratio by weight of disintegrants and binders is in the range from 10:1 to 1,25:1, preferably in the range from 8:1 to 1,5:1, more preferably in the range from 6:1 to 2:1 and most preferred in the range from 4:1 to 2,5:1.

9. Pharmaceutical composition according to any of the preceding claims, comprising sodium croscarmellose as disintegrant and lactose and/or microcrystalline cellulose (MCC) as binder(s), wherein the ratio of disintegrant and binder(s) is in the range from 4:1 to 2,5:1.

10. Pharmaceutical composition according to any of the preceding claims, comprising
(a) a granular phase comprising
• from 10,0% to 25,0% by weight of lactose and from 20,0% to 70,0% of microcrystalline cellulose (MCC);
• from 1,5% to 3,0% by weight of hydroxypropylmethylcellulose (HPMC)
• from 2,0% to 7,0% by weight of croscarmellose sodium
• between 0,1% and 1,5% by weight of colloidal silicon dioxide and
(b) an extragranular phase comprising
• from 0,5% to 5,0% by weight of one or more magnesium stearate

11. Pharmaceutical composition according to any of the preceding claims, comprising less than 30% of lactose.

12. Pharmaceutical composition according to any of the preceding claims, **characterized in that** the pharmaceutical composition is in form of a tablet.

13. Wet granulation process for the manufacture of a pharmaceutical composition according to one of the preceding claims, comprising the steps of:
(a) Mixing of Febuxostat crystalline form F10 with one or more fillers, one or more disintegrants and optionally one or more glidants;
(b) Addition of granulation liquid comprising purified water and one or more binders and granulate to form wet granules;
(c) Optionally sieving the wet granules;
(d) Drying the wet granules and calibrating the dried granules;
(e) Blending with one or more lubricants to form the final blend which is used for production of the tablets
(f) Compressing the final blend into tablets
(g) Optionally film coating of the tablets.

14. Wet granulation process according to claim 12, comprising the steps of:
(a) Mixing of Febuxostat crystalline form F10 with lactose and microcrystalline cellulose, croscarmellose sodium and colloidal silicon dioxide;
(b) Addition of granulation liquid comprising purified water and one or more hydroxypropylmethylcellulose (HPMC) and granulate to form wet granules;
(c) Sieving the wet granules;
(d) Drying the wet granules and calibrating the dried granules;
(e) Blending with magnesium stearate to form the final blend which is used for production of the tablets
(f) Compressing the final blend into tablets
(g) Optionally film coating of the tablets.
